# EUROPÄISCHE PATENTANMELDUNG

(11) **EP 2 363 422 A1**
(43) Veröffentlichungstag der Anmeldung: **07.09.2011**
(21) Anmeldenummer: 11151611.8
(22) Anmeldetag: 21.01.2011
(51) Int. Cl.: C08F 283/06, C08F 283/12, C08L 51/08, C08G 77/46, C08K 5/54, D06M 15/647

(54) **Stickstoffhaltige silizium-organische Pfropfcopolymere**

(30) Priorität: 22.02.2010 DE 102010002180
(71) Anmelder: Evonik Goldschmidt GmbH, 45127 Essen (DE)
(72) Erfinder: Kuppert, Dirk, Dr., 63739, Aschaffenburg (DE); Ferenz, Michael, Dr., 45147, Essen (DE); Schwab, Peter, Dr., 45133, Essen (DE); Knott, Wilfried, Dr., 45355, Essen (DE); Silber, Stefan, Dr., 47804, Krefeld (DE)

(57) **Zusammenfassung**

Stickstoffhaltige silizium-organische Pfropfmischpolymere von polyalkylenoxidhaltigen Siloxanderivaten und deren Verwendung.

## Beschreibung

Die Erfindung betrifft stickstoffhaltige silizium-organische Pfropfmischpolymere, erhältlich durch einen radikalischen Pfropfungsschritt mit mindestens einem ethylenisch ungesättigten Monomer, wobei mindestens ein Monomer mindestens eine quaternierbare oder quaternäre stickstoffhaltige Funktionalität enthält, in Gegenwart von polyalkylenoxidhaltigen Siloxanderivaten, wobei die polyalkylenoxidhaltigen Siloxanderivate selbst frei von ethylenischen Doppelbindungen sind und deren Verwendung.

Die Verwendung der erfindungsgemäßen silizium-organischen Pfropfmischpolymere umfasst dabei die Behandlung von Textilfasern, die Verwendung dieser Polymere als Weichmacher für Gewebe, Non-wovens und/oder Fasern aus natürlichen und/oder synthetischen Rohstoffen, die Verwendung dieser Polymere in Zusammensetzungen zum Waschen von Geweben, insbesondere zum Waschen und Reinigen von Textilien, die Reinigung und Pflege harter Oberflächen, den Einsatz zur Bautenhydrophobierung, als Verlaufs und/oder Netzmittel in Lacken und Farben, als Trennmittel und in kosmetischen Formulierungen. Weiterhin betrifft die Erfindung die Verwendung von Zusammensetzungen, die die Pfropfmischpolymere enthalten.

In den letzten Jahrzehnten haben sich industriell erzeugte Silicone zu einer bedeutenden und vielfältigen Produktgruppe entwickelt, die in fast allen Industriesektoren eine wichtige Rolle spielt und sich durch stetes Wachstum auszeichnet. Besonders die organomodifizierten Silicone haben durch ihre vielfältigen Gestaltungsmöglichkeiten dazu beigetragen, eine große Mannigfaltigkeit von Produkttypen zu ermöglichen und damit eine Vielzahl von Anwendungen zu erschließen.

Aufgrund der großen wirtschaftlichen Bedeutung ist eine Reihe von Methoden entwickelt worden, um derartige organomodifizierte Siloxane herzustellen. Dazu ist eine Verknüpfung von radikalischer Polymerisation und Siliconchemie aus vielerlei Hinsicht erstrebenswert. Die Vorteile der radikalischen Polymerisation liegen in der Vielzahl der einsetzbaren, auch in industriellem Maßstab verfügbaren Monomeren, in der hohen Toleranz gegenüber funktionellen Gruppen, einschließlich Carboxyl-, Hydroxyl-, Amino- und Epoxy-Funktionen, im relativ geringen experimentellen Aufwand und den milden sowie unempfindlichen Reaktionsbedingungen. Allerdings ist das direkte Aufpfropfen organischer Olefine auf Dialkylsiloxane trotz Offenbarung in der Literatur aus thermodynamischer Sicht und aufgrund mangelnder Verträglichkeiten sehr ungünstig und führt überwiegend zur Bildung von Homopolymeren ohne chemische Bindung an den Siloxanrücken.

Als Pfropfgrundlage eignen sich aber sehr wohl mit Polyether modifizierte Silicone, da die Ethergruppen des Polyethergerüsts durch Radikale erheblich leichter angreifbar sind. Somit können auf polyalkylenoxidhaltigen Siloxanderivaten durch Wasserstoffabstraktion Radikale erzeugt werden, von denen aus durch Addition an entsprechende vinylische Monomere eine Polymerkette gepfropft werden kann. Dies wird in DE-A-1 645 569 für kammartige Strukturen beschrieben. Die DE-A-1 645 569 offenbart ausschließlich die Anwendung dieser Propfpolymere als Stabilisatoren für Polyurethanschäume.

Polysiloxane mit quaternären Aminogruppen und deren Anwendung als Textilweichmacher sind aus der Patentliteratur bekannt. So werden zum Beispiel in der DE-AS 14 93 384 Strukturen beschrieben, bei denen Siloxane seitenständig mit Ammoniumgruppen modifiziert sind. Dabei erfolgt die Herstellung durch die Umsetzung von Siliziumwasserstoffverbindungen mit einem olefinischen Epoxid in Anwesenheit eines Platinkatalysators zu einer epoxydierten Siliziumverbindung, die in Anwesenheit eines alkoholischen Lösungsmittels mit einem sekundären Amin umgesetzt wird.

Die Patenschrift EP 0 282 720 beschreibt Strukturen, bei denen die quaternären Funktionen endständig an das Siloxan gebunden sind. Derartige Verbindungen bieten Vorteile hinsichtlich ihrer Wirkung als Textilweichmacher. So führen sie auf Textilien zu einem sehr angenehmen Griff. Dies lässt sich auf das nicht modifizierte Siloxan-Rückgrad zurückführen. Die Herstellung erfolgt dabei über die Umsetzung von endständig mit Epoxygruppen modifizierten Siloxanen mit Diaminen.

Der Nachteil der in der Patenschrift EP 0 282 720 beschriebenen Strukturen ist, dass der Modifikationsgrad maximal zwei beträgt. Wird ein Textil mit derartigen Verbindungen behandelt, so erhält es zwar einen guten Weichgriff, jedoch ist das Siloxan aufgrund seiner geringen Substantivität leicht wieder von dem entsprechendem Textil entfernbar, wie zum Beispiel durch Waschvorgänge. Es ist jedoch wünschenswert, dass das Siloxan auch nach der Wäsche auf dem Textil verbleibt und somit der Weichgriff nicht verloren geht.

Aus der DE-OS 33 40 708 sind polyquaternäre Polysiloxan-Polymere bekannt. Polyquaternäre Polysiloxan-Polymere dieses Typs weisen die oben beschriebenen Nachteile nicht auf. Der praktischen Verwendung dieser Verbindungen steht jedoch deren aufwendiges Herstellverfahren entgegen. Die Verbindungen sind nur in wirtschaftlich nicht vertretbaren Ausbeuten von 60 % der Theorie herstellbar.

Die WO 02/31256 offenbart Polyorganosiloxane mit mindestens einer quaternären Gruppe, umfassend mindestens ein Stickstoffatom, und mindestens eine weitere polare Einheit. Darüber hinaus offenbart die WO 02/31256 die Verwendung wässriger Dispersionen solcher Polyorganosiloxane zur Behandlung von Fasern. Die Polyorganosiloxane werden nach bekannten Reaktionen durch Äquilibrierung geeigneter Startmaterialen gewonnen. Nachteilig an der Synthese ist, dass der letzte Schritt der Synthese immer die Quaternisierung von einem oder mehreren Stickstoffatomen sein muß.

Weichmacherformulierungen auf Basis von Polysiloxan-Polymeren des Standes der Technik ist weiterhin gemein, dass schon nach einmaligem Waschen eines mit ihnen ausgerüsteten Textils die weichmachende Eigenschaft weitestgehend verloren geht. In Bezug auf die Behandlung von Textilfasern besteht ein gewünschtes Eigenschaftsprofil darin, einen sehr guten hydrophilen Weichgriff sowie eine erhöhte Permanenz auf Textilien zu erreichen. Darüber hinaus sind eine hohe Sprungelastizität und eine verbesserte Entknitterungseigenschaft einer so ausgerüsteten Ware als weitere positive Eigenschaften wünschenswert.

Ein weiteres wichtiges Anwendungsfeld quaternärer Polysiloxan-Polymeren ist die Reinigung und Pflege harter Oberflächen im privaten und gewerblichen Bereich.
Diese Prozesse erfordern zum Teil komplexe Formulierungen und vorgegebene geregelte Arbeitsabläufe. So besteht z.B. das Waschen von Fahrzeugen in Autowaschanlagen in der Regel aus mehreren nacheinander ablaufenden Vorgängen, die genau aufeinander abgestimmt werden müssen. Diese Abstimmung umfasst u.a. die richtige Wahl der chemischen Formulierungen die Einhaltung der Einwirkungszeiten, die Mechanik der Reinigung und die Temperaturwahl; siehe dazu auch F. Müller, J. Peggau, S. Arif, Special Purpose Cleaning Formulations: Auto Care, in Handbook of Detergents, Part D: Formulation, M. Showell, ed. CRC Press, Boca Raton 2006, pp. 261 - 278.

Die eigentliche Reinigung, die in eine Vor- und Hauptwäsche unterteilt ist und die aus verschiedenen Grundformulierungen bestehen kann, beinhaltet die Entfernung der festen, unlöslichen Schmutzpartikel auf der Fahrzeugoberfläche. Dafür gibt es eine große Anzahl an Formulierungen für die unterschiedlichsten Reinigungsverfahren. Diese Formulierungen bestehen normalerweise aus anionischen Tensidsystemen, die zusammen mit basischen oder sauren Komponenten die für die Reinigung notwendige Tensidaktivität einbringen.

An diese Reinigung schließt sich der Spülvorgang an, bei dem Reinigungsmittelreste entfernt werden müssen. Dieser Schritt dient der Vorbereitung für die Anwendung eines geeigneten Trocknungsmittels, das vor der abschließenden Gebläsetrocknung das Fahrzeug hydrophobieren und so den verbleibenden Wasserfilm leichter entfernen kann. Das Spülen ist deshalb wichtig, da Trocknungsmittel einen kationischen Charakter haben und sich sonst nach der Anwendung anionischer Reinigungsformulierungen schwerlösliche Salze bilden würden, die auf dem Fahrzeug zu unansehnlichen Flecken führen und so weder zum gewünschten Glanzeffekt noch zur Hydrophobierung führen.

Kationische Tenside, bilden bei Anwendungen, wo Substantivität gefordert ist, d.h. ein Verbleiben der oberflächenaktiven Verbindung auf dem behandelten Gut, die wesentlichen Inhaltsstoffe dieser Formulierungen. Wie bei Anwendungen im Bereich Weichspüler oder Textilausrüstung so findet diese Stoffklasse auch bei Trockneranwendungen in Autowaschanlagen ihre Verbreitung.

Da auch Fahrzeuglacke, wie die meisten Oberflächen, ein negatives elektrisches Potential aufweisen, breiten sich nach dem Aufsprühen der Trocknungsmittelformulierung die kationischen Tenside auf dem Fahrzeug aus und verdrängen den vorhandenen Wasserfilm. Dieser Vorgang, der als "Aufriss" bezeichnet wird, resultiert in einer Assoziation des Wasserfilmes zu Tropfen. Diese Tropfen laufen dann sowohl durch die eigene Schwerkraft als auch durch Benutzung eines Gebläses im letzten Schritt der Autoreinigung vom Fahrzeug herunter.

Die Formulierung von Trocknungshilfen für die automatische Fahrzeugreinigung stellt den Formulierer vor besondere Aufgaben. So muss die Formulierung nicht nur einen spontanen Wasseraufriss erzeugen, sie soll auch zu einer schnellen Trocknung und einem lang anhaltenden Glanz führen. Wichtig ist dabei die richtige Anwendungskonzentration, die bei etwa 0,1 bis 0,3 % liegen sollte. Ist die Konzentration zu niedrig, so wird der Wasserfilm nicht aufreißen, ist sie zu hoch so bildet sich auf der Fahrzeugoberfläche eine schmierige, fettige Schicht, die nicht mehr zum gewünschten Glanzeffekt führen kann.

Die Formulierung soll auch bei tiefen Temperaturen klar und ohne Ausfällungen bleiben. Darüber hinaus muss das Produkt eine große Wasserhärtetoleranz besitzen um sowohl im harten und weichem als auch wieder aufbereitetem Wasser nicht zu Trübungen zu führen. Eventuell verwendete Wachse, Öle oder andere, nicht mit Wasser mischbare Pflegemittel, die auf der Oberfläche bleiben sollen, müssen emulgiert werden.

Eine Grundformulierung für einen Trockner besteht in der Regel aus quaternären Ammoniumverbindungen, sogenannten Quats. Dabei kommen heute fast ausschließlich umweltfreundliche Esterquats oder Imidazolinquats zur Anwendung, bei denen die Fettkette hauptsächlich aus Ölsäure besteht. Da Quats meistens nicht wasserlöslich sind, erleichtern diese hochungesättigten Fettketten die Formulierung in wässrigen Systemen.

Neben Quats sind noch Rohstoffe mit emulgierenden Eigenschaften notwendig, um das o.a. Anforderungsprofil zu gewährleisten.

Im Zuge der Beschleunigung des Betriebs von Autowaschstrassen sind diverse Versuche unternommen worden, den relativ langwierigen Aufrissprozess zu beschleunigen. Es wurden zum Beispiel Siliconverbindungen des quaternären Typs wie in DE 101 07 772 beschrieben getestet, jedoch ohne Erfolg. Da einer der geschwindigkeitsbestimmenden Schritte in der Autowaschstrasse der Ablauf des Trockners ist, wird eine Beschleunigung den Durchsatz an Fahrzeugen in der Waschstrasse erhöhen und damit Wartezeiten für Kunden reduzieren und die Anlageneffizienz erhöhen.

Die WO 99/04750 offenbart die Verwendung von Polymeren, die wasserlöslich oder wasserdispergierbar sind oder die, für den Fall, dass sie aus Monomeren mit neutralisierbaren Resten bestehen, in neutralisierter Form wasserlöslich oder wasserdispergierbar sind, die erhaltlich sind indem man (a) ethylenisch ungesättigte Monomere in Gegenwart von (b) polyalkylenoxid-haltigen Silikonderivaten radikalisch polymerisiert, für kosmetische Formulierungen.

Es besteht daher ein Bedarf an stickstoffhaltigen Polysiloxan-Polymeren und insbesondere an solchen Polysiloxan-Polymeren mit quaternären Aminogruppen, welche sich in guten Ausbeuten herstellen lassen, vielseitig einsetzbar sind und darüber hinaus verbesserte Eigenschaften als die Polymere des Standes der Technik besitzen, bzw. die einen oder mehrere Nachteile des Standes der Technik nicht aufweisen. Insbesondere sollte auch ein wirtschaftliches Verfahren zu deren Herstellung angewandt werden, dass nicht zur Bildung von unbrauchbaren Nebenprodukten (z.B.: Koagulat bei der Emulsionspolymerisation) führt.

Aufgabe der vorliegenden Erfindung war es daher, stickstoffhaltige Polysiloxan-Polymere aufzufinden und insbesondere solche Polysiloxan-Polymere mit quaternären Aminogruppen, die einen oder mehrere Nachteile des Standes der Technik nicht mehr aufweisen.

Überraschenderweise wurde nun gefunden, dass die Verwendung stickstoffhaltiger silizium-organischer Pfropfmischpolymere, erhältlich durch einen radikalischen Pfropfungsschritt mit mindestens einem ethylenisch ungesättigten Monomeren, wobei mindestens ein Monomer mindestens eine quaternierbare oder quaternäre stickstoffhaltige Funktionalität enthält, in Gegenwart von polyalkylenoxidhaltigen Siloxanderivaten, die frei von ethylenisch ungesättigten Gruppen sind, in besonderer Weise die Aufgabe lösen kann.

Gegenstand der vorliegenden Erfindung sind daher stickstoffhaltige silizium-organische Pfropfmischpolymere, erhalten durch einen radikalischen Pfropfungsschritt mit mindestens einem ethylenisch ungesättigten Monomeren, wobei mindestens ein Monomer mindestens eine quaternierbare oder quaternäre stickstoffhaltige Funktionalität enthält, in Gegenwart von polyalkylenoxidhaltigen Siloxanderivaten, die frei von ethylenisch ungesättigten Gruppen sind.
Dabei kann die quaternierbare oder quaternäre stickstoffhaltige Funktionalität eine cyclische oder acyclische Aminfunktionalität sein.

Weitere Gegenstände der Erfindung ist auch ein Verfahren zur Herstellung dieser Pfropfmischpolymere sowie deren Verwendung.
Besonders geeignet zur Lösung der Aufgabe sind dabei silizium-organische Pfropfmischpolymere, die mindestens eine permanent quaternäre, dass heißt, positive geladene Stickstofffunktionalität tragen.

Ganz besonders geeignet zur Lösung der Aufgaben sind dabei silizium-organische Pfropfmischpolymere, die mindestens eine permanent quaternäre Stickstofffunktionalität aufweisen, erhältlich durch die radikalische Pfropfpolymerisation von mindestens einem ethylenisch ungesättigten Monomeren mit mindestens einer quaternären stickstoffhaltigen Funktionalität, in Gegenwart von polyalkylenoxidhaltigen Siloxanderivaten.

Die stickstoffhaltigen silizium-organischen Pfropfmischpolymere eignen sich für verschiedenste, wie die z. B. oben aufgeführten, Anwendungen, in denen organisch modifizierte Polyethersiloxane eingesetzt werden.

Die polyalkylenhaltigen Siloxane als Pfropfgrundlage, in deren Gegenwart die radikalische Umsetzung erfolgt, werden ausgewählt aus Polyethersiloxanen der Formel (I), 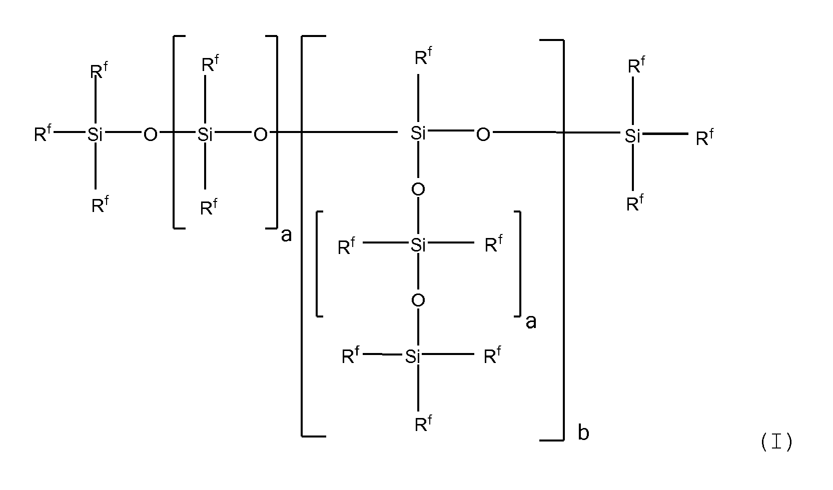 mit
b eine Zahl von 0 bis 10, vorzugsweise < 5 und bevorzugt 0 ist,
a eine Zahl von 1 bis 500, vorzugsweise 1 bis 250 und bevorzugt 1 bis 100 ist,
R^{f} gleiche oder verschiedene Reste R¹ oder R² sind, mit der Maßgabe, dass mindestens ein Rest R^{f} ein Rest R² ist,
R¹ organische Reste, ausgewählt aus linearen oder verzweigten Alkyl-, Halogenalkyl-, Aryl-, Alkylaryl- oder Arylalkylresten mit 1 bis 30 Kohlenstoffatomen, vorzugsweise 2 bis 6 C-Atome, bei Arylresten vorzugsweise Phenyl, wobei die Reste gegebenenfalls durch ein oder mehrere Sauerstoff- und/oder Stickstoff-Atome unterbrochen sein können und/oder am Ende des Restes gegebenenfalls eine -OC(O)CH₃ Gruppe aufweisen können,
R² eine Gruppe der Formel A_{α}-B_{β}-K_{χ}-D_{δ}-E_{ε}- L_{λ}darstellt, mit
   α 1,
   β , χ, δ und ε 0 oder 1,
   λ 1und
   α + β + χ ≥ 1, wobei
   A ein Sauerstoffatom oder eine CH₂-Gruppe ist,
   B eine Gruppe der allgemeinen Formel (II) 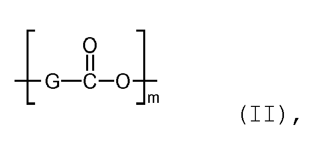 ist, wobei
   m eine ganze Zahl von 0 bis 30 ist und
   G eine zweiwertige Gruppe, ausgewählt aus linearen oder verzweigten, gesättigten Alkyl-, Aryl-, Alkylaryl-oder Arylalkylgruppen mit 1 bis 20 Kohlenstoffatomen sein kann,
   K eine -CH₂-Gruppe oder ein zweiwertiger Rest, ausgewählt aus linearen oder verzweigten, gesättigten Alkyl-, Aryl-, Alkylaryl- oder Arylalkyl-Oxygruppen mit 1 bis 20 Kohlenstoffatomen oder eine Gruppe der Formel -CH₂-O- (CH₂) ₄-O- ist,
D eine Gruppe der allgemeinen Formel (III)
   - (C₂H₄O)ₙ(C₃H₆O)ₚ(C₁₂H₂₄O)q(C₈H₈O)ᵣ(C₄H₈O)ₛ- (III)
   wobei die Indices n, p, q, r und s voneinander unabhängige ganze Zahlen von 0 bis 100 sind,
   und wobei die Summe aus n, p, q, r und s ≥ 1, vorzugsweise 2 bis 250, bevorzugt 5 bis 150 und besonders bevorzugt 10 bis 80 ist, und falls mehr als einer der Indices n, p, q, r, s > 0 ist, die allgemeine Formel (III) ein statistisches Oligomer oder ein Blockoligomer sein kann. Vorzugsweise ist R² ein Polyetherrest der Formel (III) bei dem n und/oder p jeweils ≥ 3, vorzugsweise 3 bis 100 und bevorzugt 5 bis 50 ist. Bevorzugt ist R² ein Polyetherrest der Formel (III) bei dem n und p jeweils ≥ 3, vorzugsweise 3 bis 100 und bevorzugt 5 bis 80 ist und q und/oder r, s 0, vorzugsweise q, r und s 0, ist
E eine Gruppe der allgemeinen Formel (IV) 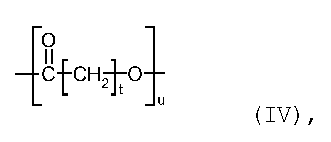 ist, wobei
u eine ganze Zahl von 0 bis 5 ist und
t, falls u > 0 ist, gleich oder verschieden sein kann und 3, 4 oder 5 darstellt und
L ausgewählt ist aus der Gruppe umfassend Wasserstoffatome, lineare oder verzweigte, gesättigte Alkyl-, Aryl-, Alkylaryl- oder Arylalkylgruppen mit 1 bis 12 Kohlenstoffatomen, bevorzugt mit 1 bis 10, oder Acetoxy-Gruppen.

Besonders bevorzugt sind Polyethersiloxane der Formel (I), bei denen A = -CH₂-, α - 1, β = 0, χ = 1 ist. Ganz besonders bevorzugt sind Polyethersiloxane der Formel (I), bei denen A = -CH₂-, α = 1, β = 0, χ = 1 und K = -CH₂-CH₂-O- ist.

Die erhaltenen Polyethersiloxane können geradkettig (b = 0) oder verzweigt sein (0 < b ≤ 10). Die Werte von a und b sind als durchschnittliche Werte zu verstehen, da die eingesetzten Polysiloxane nicht nur als Reinstoff sondern auch in Form von äquilibrierten Gemischen vorliegen können.

Es ist dem Fachmann bekannt, dass die Verbindungen aufgrund ihrer polymeren Natur in Form eines Gemisches mit einer im Wesentlichen durch statistische Gesetzte geregelten Verteilung vorliegen. Die Werte für alle Indices stellen deshalb Mittelwerte dar. Vorzugsweise werden solche äquilibrierten Gemische von Poly(ether)siloxanen eingesetzt.

Die Herstellung dieser ethylenisch gesättigten Polyethersiloxane kann mit für den Fachmann geläufigen Methoden für den Fall von A in Formel (I) ungleich Sauerstoff durch Hydrosilylierung von Wasserstoffsiloxanen der allgemeine Formel (V) mit Alkenylpolyethern in Gegenwart von Platin oder Rhodium Katalysatoren, wie z. B. in der EP-A-0 659 803 beschrieben, erfolgen.

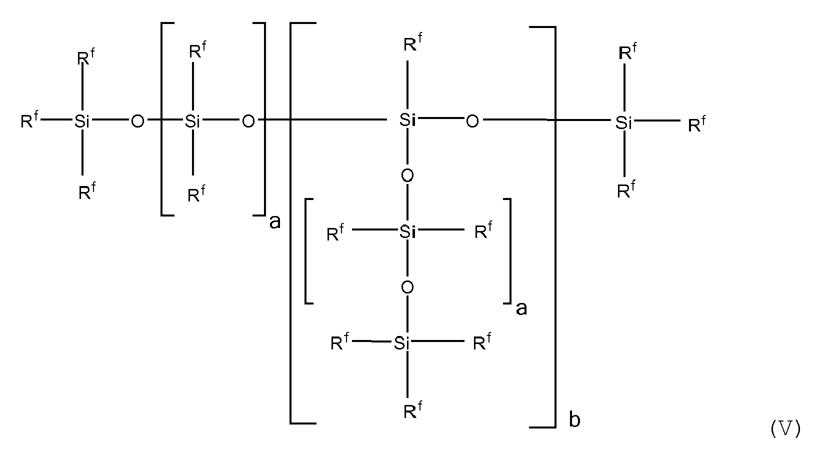

Dabei haben in Formel (V) a, b und R^{f} die Bedeutung wie in Formel (I) mit der Ausnahme, dass R² = H ist.

Bevorzugt eingesetzt werden dabei Alkenylpolyether ausgewählt aus Alkenylpolyethern der Formel (VI)

CH₂=CR³-Q- (C₂H₄O) ₙ (C₃H₆O) p (C₁₂H₂₄O) q (C₈H₈O) , ᵣ (C₄H₈O) ₛL (VI)

wobei
R³ oder Methyl ist,
Q ein zweiwertiger gegebenenfalls verzweigter Kohlenwasserstoffrest mit 1 bis 18 C-Atomen, vorzugsweise 1 bis 4 C-Atomen und bevorzugt einem C-Atom ist,
L ein H-Atom oder ein einwertiger linearer oder verzweigter organischer Rest wie Alkyl, Aryl, Alkylaryl, Arylalky oder Acetoxy, vorzugsweise mit 1 bis 20, bevorzugt 1 bis 10 Kohlenstoffen, die Indices
n, p, q, r und voneinander unabhängige ganze Zahlen von 0 bis 100 sind, die Summe aus
n, p, q, r und s ≥ 1 und falls mehr als einer der Indices n, p, q, r, s > 0 ist, die allgemeine Formel (IV) ein statistisches Oligomer oder ein Blockoligomer sein kann, eingesetzt wird.

Vorzugsweise beträgt die Summe aus n, p, q, r und s 2 bis 250, bevorzugt 5 bis 150 und besonders bevorzugt 10 bis 80. Bevorzugt sind solche Alkenylpolyether (VI), bei denen n und/oder p jeweils ≥ 3, vorzugsweise 3 bis 100 und bevorzugt 5 bis 50 ist. Besonders bevorzugt sind solche Alkenylpolyether (VI) bei denen n und p jeweils ≥ 3, vorzugsweise 3 bis 100 und bevorzugt 5 bis 80 ist und q und/oder r und/oder s 0, vorzugsweise q und r und s = 0, ist.

Die nach Formel (VI) beschriebenen Polyether können z. B. aus einem Startalkohol mit einer alpha-ständigen Kohlenstoff-Kohlenstoff Doppelbindung durch Anlagerung von Monomeren gewonnen werden. Geeignete Monomeren sind Ethylenoxid, Propylenoxid, Verbindungen aus der Gruppe Tetrahydrofuran, 1,2-Epoxybutan, 2,3-Epoxybutan, Dodecyloxid, Styroloxid und/oder Methylstyroloxid und Mischungen daraus. Dabei kann die Verteilung der Monomere beliebig gewählt sein, so dass ein statistisches Oligomer oder ein Blockpolymer erhalten werden kann.

Die Herstellung von Polyethersiloxanen nach Formel (I), wobei A ein Sauerstoff ist, kann ebenfalls mit den für den Fachmann geläufigen Methoden durch dehydrogenative Kondensation von Hydroxygruppen-terminierten Polyethern mit Wasserstoffsiloxanen erhalten werden.
Vorzugsweise wird die dehydrogenative Kondensation in Gegenwart eines Katalysators durchgeführt. Geeignete Katalysatoren für die dehydrogenative Kondensation sind beispielsweise NaOH, KOH, Tetramethylammoniumhydroxid, Alkalifluoride, Erdalkalifluoride, Borkatalysatoren wie Tris(pentafluorphenyl)boran, Carbonsäuren und/oder Carboxylate oder deren Mischungen. Die katalytische dehydrogenative Kondensation wird beispielsweise in den Schriften E P-A-1 460 098, DE-A-103 12 636 und DE-A-10359 764, beschrieben.
Als Pfropfgrundlage geeignete Siliconderivate sind weiterhin die unter den INCI Namen Dimethicone Copolyole oder Silicontenside bekannten und kommerziell erhältlichen Verbindungen, wie zum Beispiel die unter dem Markennamen Abil® oder Tegoprer® der Firma Evonik Goldschmidt GmbH gehandelten Verbindungen.

Monomere die mit einer durch Radikale initiierten Reaktion polymerisiert werden können sind bevorzugt. Der Begriff ethylenisch ungesättigt bedeutet, dass die Monomere zumindest eine polymerisierbare Kohlenstoff-Kohlenstoff Doppelbindung besitzen, die mono-, di-, tri-, oder tetrasubstituiert sein kann.

Jegliche monomere, ethylenisch ungesättigte Verbindung und jegliches polymeres Olefin mit mindestens einem Rest an Ungesättigtkeit (wie Polymere des Butadiens oder Isoprens oder jegliche Art von Macromonomeren, einschließlich solcher die Siloxanketten enthalten) und mindestens eine stickstoffhaltige Funktionalität besitzt, die quaterniert werden kann, können in der Pfropfpolymerisation zur Herstellung der erfindungsgemäßen Pfropfmischpolymere verwendet werden.
Die genannten Monomere, die mindestens eine stickstoffhaltige Funktionalität besitzt, die quaterniert werden kann, können auch in Mischungen mit stickstofffreien Monomeren pfropfpolymerisiert werden.

Ganz besonders bevorzugt können jegliche monomere, ethylenisch ungesättigte Verbindung und jegliches polymeres Olefin mit mindestens einem Rest an Ungesättigtkeit (wie Polymere des Butadiens oder Isoprens oder jegliche Art von Macromonomeren, einschließlich solcher die Siloxanketten enthalten) und mindestens eine quaternäre stickstoffhaltige Funktionalität aufweisen in der Pfropfpolymerisation zur Herstellung der erfindungsgemäßen Pfropfmischpolymere verwendet werden.
Als geeignete polymerisierbare Monomere können bevorzugt ethylenisch ungesättigte Monomere verwendet werden. Dabei kann entweder ein einzelnes Monomer oder Kombinationen von zwei oder mehr Monomeren verwendet werden unter der Voraussetzung, dass mindestens ein Monomer eine stickstoffhaltige Funktionalität besitzt, die quaterniert werden kann. Mit polymerisierbar ist gemeint, dass die verwendeten Monomere unter Verwendung irgendeiner konventionellen synthetischen Methode polymerisiert werden können.

Zusätzlich zu mindestens einem Monomer mit mindestens einer stickstoffhaltigen Funktionalität können auch stickstofffreie Verbindungen im Pfropfungschritt eingesetzt werden, die durch die folgende allgemeine Formel (VII) beschrieben werden: 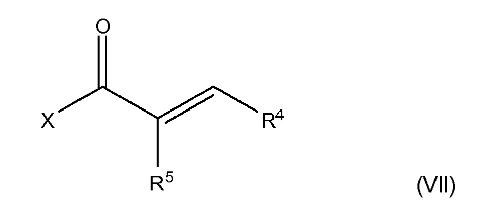 wobei R⁵ und R⁴ unabhängig voneinander ausgewählt sind aus der Gruppe enthaltend -H, C₁-C₈ linear- oder verzweigtkettige Alkylketten, Methoxy, Ethoxy, 2-Hydroxyethoxy, 2-Methoxyethoxy und 2-Ethoxyethyl,
X ausgewählt ist aus der Gruppe der Reste -OH, -OM, -OR⁶, wobei der Rest R⁶ ausgewählt werden kann aus der Gruppe bestehend aus -H, C₁-C₄₀ linear- oder verzweigtkettige Alkylreste, 2-Hydroxyethyl, 2-Methoxyethyl, 2-Ethoxyethyl, Hydroxypropyl, Methoxypropyl oder Ethoxypropyl.
M ist ein Kation ausgewählt aus der Gruppe bestehend aus: Na⁺, K⁺, Mg⁺⁺ ,Ca⁺⁺ ,Zn⁺⁺, NH₄⁺, Alkylammonium, Dialkylammonium, Trialkylammonium und Tetraalkylammonium.

Repräsentative aber nicht limitierende Beispiele von geeigneten Monomeren sind zum Beispiel Acrylsäure und deren Salze, Ester und Amide. Die Salze können von jedem beliebigen nicht toxischen Metall, Ammonium oder substituierten Ammoniumgegenionen abgeleitet sein.

Die Ester können abgeleitet sein von C₁-C₄₀ linearen, C₃-C₄₀ verzweigtkettigen, oder C₃-C₄₀ carbocyclischen Alkoholen, von mehrfachfunktionellen Alkoholen mit 2 bis etwa 8 Hydroxylgruppen wie Ethylenglycol, Hexylenglycol, Glycerin, und 1,2,6-Hexantriol, von Aminoalkoholen oder von Alkoholethern wie Methoxyethanol, Ethoxyethanol oder Polyalkylenglykolen, wie z.B. Polyethylenglykolen.

Ebenfalls verwendbare Monomeren sind substituierte Acrylsäuren sowie Salze, Ester und Amide davon, wobei die Substituenten an den Kohlenstoffatomen in der zwei oder drei Positionen der Acrylsäure stehen, und unabhängig voneinander ausgewählt sind aus der Gruppe bestehend aus C₁-C₄ Alkyl, -CN, COOH besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Andere geeignete Monomere sind Vinyl- und Allylester von C₁-C₄₀ linearen, C₃-C₄₀ verzweigtkettigen oder C₃ - C₄₀ carbocyclische Carbonsäuren (z.B.: Vinylacetat, Vinylpropionat, Vinylneononanoat, Vinylneoundekansäure oder t-Butylbenzoesäure-vinylester); Vinyl- oder Allylhalogenide, bevorzugt Vinylchlorid und Allylchlorid, Vinylether, bevorzugt Methyl-, Ethyl-, Butyl-, oder Dodecylvinylether, Vinylformamid, Vinylmethylacetamid, Vinylamin; Vinyllactame, bevorzugt Vinylpyrrolidon und Vinylcaprolactam, Vinyl- oder Allylsubstituierte heterocyclische Verbindungen, bevorzugt Vinylpyridin, Vinyloxazolin und Allylpyridin.
Darüber hinaus geeignete und mitverwendbare stickstofffreie Monomere sind Vinylidenchlorid; und Kohlenwasserstoffe mit mindestens einer Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomeren.

Zusätzlich zu den oben genannten Monomeren können als Monomere sogenannte Makromonomere wie zum Beispiel silikonhaltige Makromonomere mit ein oder mehreren radikalisch polymerisierbaren Gruppen oder Alkyloxazolinmakromonomere eingesetzt werden wie sie zum Beispiel in der EP 408 311 beschrieben

Des Weiteren können fluorhaltige Monomere wie sie beispielsweise in der EP 558423 beschrieben sind, sowie vernetzend wirkende oder das Molekulargewicht regelnde Verbindungen in Kombination oder alleine eingesetzt werden.

Als stickstoffhaltige Monomere werden bevorzugt eingesetzt N,N-Dialkylaminoalkylacrylate- und methacrylate und N-Dialkylaminoalkylacryl- und -methacrylamide der allgemeinen Formel (VIII) 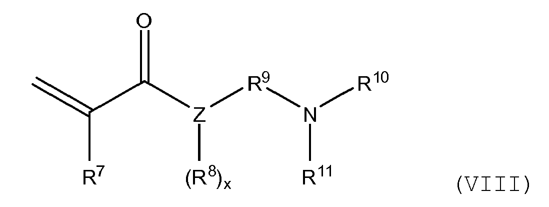 mit R⁷ = H, Alkyl mit 1 bis 8 C-Atomen, R⁸ = H, Methyl, R⁹ = Alkylen mit 1 bis 24 C-Atomen, optional substituiert durch Alkyl, R¹⁰ und R¹¹ unabhängig voneinander = C₁-C₄₀ Alkylrest, Z = Stickstoff für x = 1 oder Sauerstoff für x = 0 Die Amide können unsubstituiert, N-Alkyl oder N-alkylamino monosubstituiert, oder N,N-dialkylsubstituiert oder N,N-dialkylamino disubstituiert, worin die Alkyl- oder Alkylaminogruppen von C₁-C₄₀ linearen, C₃-C₄₀ verzweigtkettigen, oder C₃-C₄₀ carbocyclischen Einheiten abgeleitet sind. Zusätzlich können die Alkylaminogruppen quaternisiert werden.

Besonders bevorzugte Monomere der Formel (VIII) sind N,N-Dimethylaminomethyl (meth)acrylat, N,N-Diethylaminomethyl (meth)acrylat, N,N-Dimethylaminoethyl(meth)acrylamid, N,N-Diethylaminoethyl(meth)acrylamid, N,N-Dimethylaminopropyl (meth)acrylat, N,N-Diethylaminopropyl(meth)acrylat, N,N-Dimethylaminopropyl(meth)acrylamid, N,N-Diethylaminopropyl (meth)acrylamid.

Weiterhin sind N-Vinylimidazole der allgemeinen Formel (IX) geeignet stickstoffhaltige Monomeren, worin R¹² bis R¹⁴ unabhängig voneinander für Wasserstoff, C₁-C₄-Alkyl oder Phenyl steht:

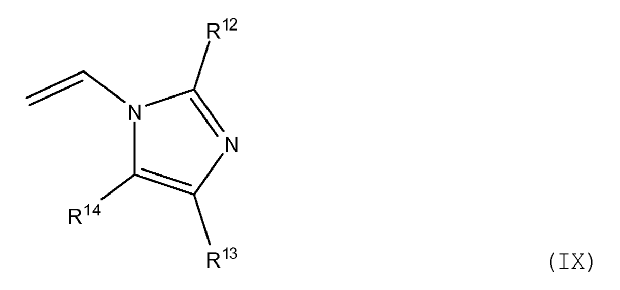

Weitere geeignete stickstoffhaltige Monomeren sind Diallylamine der allgemeinen Formel (X) 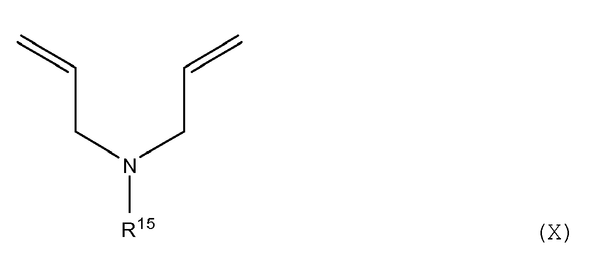 mit R¹⁵ = C₁ bis C₂₄ Alkyl.

Monomere, mit einem basischen Stickstoffatom, können dabei auf folgende Weise quaternisiert werden: Zur Quaternisierung der Amine eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid.

Weitere geeignete Quaternierungsmittel sind Dialkylsulfate, insbesondere Dimethylsulfat oder Diethylsulfat. Die Quaternierung der basischen Amine kann auch mit Alkylenoxiden, wie z.B. Ethylenoxid, in Gegenwart von Brönstedsäuren erreicht werden.
Bevorzugte Quaternierungsmittel sind: Methylchlorid, Dimethylsulfat oder Diethylsulfat.

Besonders bevorzugt eingesetzte Monomeren sind Monomere, die schon mindestens eine quaternäre stickstoffhaltige Gruppe aufweisen, insbesondere Monomeren der allgemeinen Formel (XI) 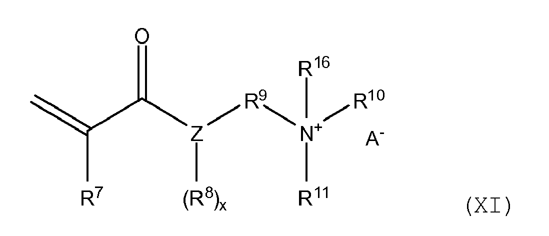 mit der Bedeutung von R⁷, R⁸ R⁹ ,R¹⁰, R¹¹ , ,Z und x wie in Formel (VIII), R¹⁶ = C₁-C₄₀ Alkylrest und A⁻ ein geeignetes negativ geladenes Anion, wie z.B. Fluorid, Chlorid, Bromid, Jodid, Alkylsulfate, wie z.B. Methylsulfat oder Ethylsulfat, Sulfat, Hydrogensulfat, Methansulfonat, Trifluormethansulfonat. Besonders bevorzugt sind dabei Chlorid, Methylsulfat und Ethylsulfat. Ganz besonders bevorzugt ist Chlorid.

Besonders bevorzugt eingesetzte Monomeren der Formel (XI) sind 2-Trimethylammoniumethylmethacrylatchlorid, 2-Trimethylammoniummethylacrylatchlorid, 2-Triethylammoniumethylmethacrylatchlorid, 2-Triethylammoniumethylacrylatchlorid, 3-Trimethylammoniumpropylmethacrylamidchlorid, 3-Trimethylammoniumpropylacrylamidchlorid, 3-Triethylammoniumpropylmethacrylamidchlorid, 3-Triethylammoniumpropylacrylamidchlorid.

Die Quaternisierung kann vor der Polymerisation oder nach der Polymerisation durchgeführt werden. Bevorzugt wird die Quaternierung vor der Polymerisation durchgeführt. Ganz besonders bevorzugt werden Monomeren mit mindestens einer quaternären stickstoffhaltigen funktionellen Gruppe, wie in Formel (XI) offenbart, eingesetzt.

Außerdem können die Umsetzungsprodukte von ungesättigten Säuren, wie z.B. Acrylsäure oder Methacrylsäure, mit einem quaternisierten Epichlorhydrin der allgemeinen Formel (XII) eingesetzt werden mit R¹⁷ = C₁ bis C₄₀ Alkyl und A⁻ ein geeignetes negativ geladenes Anion, wie z.B. Fluorid, Chlorid, Bromid, Jodid, Alkylsulfate, wie z.B. Methylsulfat oder Ethylsulfat, Sulfat, Hydrogensulfat, Methansulfonat, Trifluormethansulfonat. Besonders bevorzugt sind dabei Chlorid, Methylsulfat und Ethylsulfat. Ganz besonders bevorzugt ist Chlorid.

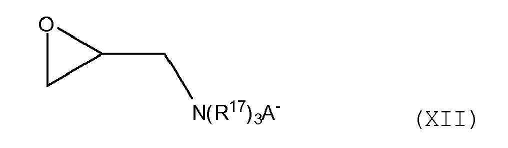

Beispiele hierfür sind zum Beispiel: (Meth)acryloyloxyhydroxypropyltrimethylammoniumchlorid und (Meth) acryloyloxyhydroxy- propyltriethylammoniumchlorid.

Die basischen Monomere können auch kationisiert werden, indem sie mit Mineralsäuren, wie z.B. Schwefelsäure, Chlorwasserstoffsäure, Bromwasserstoffsäure, Iodwasserstoffsäure, Phosphorsäure oder Salpetersäure, oder mit organischen Säuren, wie z.B. Ameisensäure, Essigsäure, Milchsäure, oder Citronensäure, neutralisiert werden.

Jegliche Kombination aus den genannten Monomeren kann in beliebigen Mischungsverhältnissen eingesetzt werden unter der Voraussetzung, dass mindestens ein Monomer mindestens eine stickstoffhaltige Funktionalität aufweist. Weiterhin erwünscht ist die Verträglichkeit der Monomeren. Insbesondere können auch solche Kombinationen gewählt werden, in denen die Monomere unterschiedliche Reaktivitäten aufweisen und somit Gradientencopolymere entstehen.

Als Regler können die üblichen dem Fachmann bekannten Verbindungen wie zum Beispiel Schwefelverbindungen (z.B.: Mercaptoethanol, 2-Ethylhexylthioglykolat, Thioglykolsäure oder Dodecylmercaptan) sowie Tribromchlormethan oder andere Verbindungen die regelnd auf das Molekulargewicht der erhaltenen Pfropfpolymerisate wirken, verwendet werden. Als Regler können gegebenenfalls auch thiolgruppenhaltige Siliconverbindungen oder thiolgruppenhaltige Alkenylpolyether eingesetzt werden. Bevorzugt werden aber siliconfreie Regler eingesetzt oder die Synthesebedienungen so eingestellt, dass keine Regler verwendet werden müssen.

Die ethylenisch ungesättigten Monomere können im Pfropfungsschritt unter Verwendung irgendeiner konventionellen, dem Fachmann bekannten synthetischen Methode radikalisch mit einer, eine Polyethergruppe aufweisende Verbindung, umgesetzt werden.

Der Pfropfungsschritt kann als Lösungspolymerisation, Emulsionspolymerisation, umgekehrte Emulsionspolymerisation, Suspensionspolymerisation, umgekehrte Suspensionspolymerisation oder als Fällungspolymerisation durchgeführt werden, ohne dass die verwendbaren Methoden darauf beschränkt sind. Die radikalische Umsetzung kann Z. B. wie in DE-1 645 569 beschrieben, auf welche ausdrücklich verwiesen wird, durchgeführt werden.

Der Pfropfungsschritt kann in Gegenwart oder bei Abwesenheit von Lösungsmitteln erfolgen. Der Pfropfungsschritt kann in Ein-, Zwei- oder Mehrphasensystemen durchgeführt werden. Wichtige Voraussetzung dabei ist lediglich die wechselseitige Löslichkeit der Reaktionspartner im entsprechenden Medium (Lösungsmittel). Wird im Pfropfungsschritt insbesondere bei der Lösungsmittelpolymerisation, ein Lösungsmittel eingesetzt, so kann dieses ein organisches Lösemittel, das eingesetzte Polysiloxan(-gemisch) bzw. der eingesetzte Alkenylpolyether oder Wasser oder Gemische davon sein.

Der Pfropfungsschritt kann unter Normaldruck, Überdruck, oder Unterdruck durchgeführt werden. Besonders bevorzugt wird der Pfropfungsschritt unter Normaldruck durchgeführt.

Ein Stoff, der unter den Reaktionsbedingungen freie Radikale bildet, ist wesentliche Voraussetzung und Bestandteil des Pfropfungsschrittes zur Herstellung der Pfropfmischpolymere. Alle Mittel, die prinzipiell geeignet sind, freie Radikale zu erzeugen, können eingesetzt werden, u. a. aber nicht ausschließlich ionisierende Bestrahlung, organische Peroxyverbindungen, Azo-Verbindungen oder anorganische Radikalbildner, wie z. B. anorganische Peroxodisulfate. Bevorzugt eingesetzt werden Azo-Verbindungen und organische Peroxyverbindungen.
Bei wasserhaltigen Reaktionsystemem oder Wasser als Lösemittel werden ganz besonders bevorzugt Alkali- und Ammoniumperoxodisulfat als Radikalbildner eingesetzt. Weiterhin können auch Redoxsysteme, wie z.B. Kaliumperoxodisulfat und Natriumhydrogensulfit, zur Erzeugung von Radikalen eingesetzt werden.

Zur besseren Dosierung oder Verträglichkeit der Radikalbildner kann es vorteilhaft sein, die Radikalbildner in Form von Lösungen dieser in einem geeigneten Lösungsmittel einzusetzen. Als geeignete Lösungsmittel können alle Lösungsmittel eingesetzt werden, die mit der radikalischen Umsetzung nicht interferieren. Vorzugsweise werden die Lösungsmittel eingesetzt, die als geeignete Lösungsmittel für die radikalische Umsetzung genannt wurden. Werden anorganische Radikalbildner eingesetzt, wird als Lösungsmittel für die Radikalbildner bevorzugt Wasser eingesetzt.

Die für die Reaktion gewählte Temperatur hängt von den zum Einsatz kommenden Radikale bildenden Verbindungen ab. Wird die Radikalbildung thermisch induziert, spielt die Halbwertszeit des Zerfalls in Primärteilchen eine entscheidende Rolle und kann so gewählt werden, dass sich in der Reaktionsmischung immer ein gewünschtes Verhältnis von freien Radikalen einstellt. Für die thermisch induzierte Radikalbildung werden vorzugsweise Temperaturen von 30 °C bis 225 °C, bevorzugt 50 bis 180 °C eingesetzt, wobei die Temperatur nach oben durch die thermische Zersetzung der Pfropfgrundlage begrenzt wird. Im Fall des Einsatzes von Redoxsystemen als Radikalbildner werden vorzugsweise Temperaturen von -30 °C bis 50 °C eingesetzt.

Als vernetzende Monomere können Verbindungen mit mindestens zwei ethylenisch ungesättigten Doppelbindungen eingesetzt werden wie zum Beispiel Ester von ethylenisch ungesättigten Carbonsäuren, wie Acrylsäure oder Methacrylsäure und mehrwertigen Alkoholen, Ether von mindestens zweiwertigen Alkoholen wie zum Beispiel Vinylether oder Allylether. Ausserdem geeignet sind geradkettige oder verzweigte, lineare oder cyclische aliphatische oder aromatische Kohlenwasserstoffe, die aber mindestens zwei Doppelbindungen vertragen, welche bei den aliphatischen Kohlenwasserstoffen nicht konjugiert sein dürfen. Ferner geeignet sind Amide der Acryl- und Methacrylsäure und N-Allylamine von mindestens zweiwertigen Aminen wie zum Beispiel (1,2-Diaminoethan, 1,3-Diaminopropan). Ferner geeignet sind Triallylamin oder entsprechende Ammoniumsalze, N-Vinylverbindungen von Harnstoffderivaten, mindestens zweiwertigen Amiden, Cyanuraten oder Urethanen.

Besonders bevorzugte Vernetzer sind beispielsweise Methylenbisacrylamid, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen die mit Ethylenoxid und/oder Propylenoxid und/oder Epichlorhydrin umgesetzt worden sind. Wie dem Fachmann geläufig ist, können aber die Molekulargewichte so eingestellt werden, dass keine Vernetzer notwendig sind.

Die erfindungsgemäßen Zusammensetzungen können Pfropfmischpolymere aufweisen, die jegliche relative Mengen ethenylisch ungesättigter Verbindungen aufgepfropft auf das Polyethersiloxan als Pfropfgrundlage enthalten. Bevorzugte Mengenverhältnisse variieren je nach Anwendung und liegen im Allgemeinen zwischen 1 und 10.000 Gew.% der jeweils zugrunde liegenden Pfropfgrundlage.
Bevorzugt beträgt die Menge des eingesetzten quaternierbaren oder quaternären Monomers 1 bis 75 Gew.-% bezogen auf das Polyethersiloxan. Besonders bevorzugt ist ein Verhältnis von 1 bis 50 Gew.-% und ganz besonders bevorzugt von 2 bis 25 Gew.-%. Werden weitere, nicht quaternäre oder nicht quaternierbare Monomere bei der radikalischen Pfropfung eingesetzt, so wird ein Verhältnis von Polyethersiloxan zu nicht quaternärem oder nicht quaternierbaren Monomeren zwischen 0,1 und 99 Gew.-% bevorzugt. Besonders bevorzugt wird ein Verhältnis von 1 bis 75 Gew.-% und ganz besonders bevorzugt ein Verhältnis von 1 bis 50 Gew.-%, unabhängig von dem Verhältnis des quaternierbaren oder quaternären Monomers und des Polyethersiloxans.

Bevorzugte Zusammensetzungen weisen solche Pfropfmischpolymere auf, die durch einen radikalischen Pfropfungsschritt in Gegenwart von polyalkylenoxidhaltigen Siloxanderivaten mit mindestens einem ethylenisch ungesättigten Monomeren das eine stickstoffhaltige Funktionalität aufweist, erhältlicht sind. Das polyalkylenoxidhaltige Siloxanderivat ist dabei bevorzugt frei von ethylenisch ungesättigten Gruppen.

Ganz besonders bevorzugte Zusammensetzungen weisen solche Pfropfmischpolymere auf, die durch einen radikalischen Pfropfungsschritt in Gegenwart von polyalkylenoxidhaltigen Siloxanderivaten mit mindestens einem ethylenisch ungesättigten Monomeren, wobei mindestens ein Monomer mindestens eine quaternierte stickstoffhaltige Funktionalität enthält, erhältlich sind.

Weiterhin ist Gegenstand dieser Erfindung die Verwendung der stickstoffhaltigen silizium-organischen Pfropfmischpolymere oder Zusammensetzungen als Weichmacher oder Weichgriffmittel für Gewebe, Non-wovens und/oder Fasern aus natürlichen und/oder synthetischen Rohstoffen, für Textilien, als Additiv in kosmetischen Anwendungen, als Bestandteil von Haarbehandlungsmitteln in Form von Shampoo, Haarspülung, Konditioniermittel, Haarspray, und/oder als Bestandteil von Haarstylinggel.

Darüber hinaus lassen sich die erfindungsgemäßen polyalkylenoxidhaltigen Siloxanderivate in Reinigungsmitteln für die Reinigung harter Oberflächen, harten lackierten oder unlackierten Oberflächen aus Glas, Keramik, Kunststoff oder Metall, und von Geschirr, in Haushalt und Gewerbe, sowie in der gewerblichen Autowäsche in Trocknungshilfsmitteln in Autowaschstraßen verwenden. Harte Oberflächen umfassen auch glatte, raue, profilierte oder unprofilierte Fliesen, Steinzeugfließen und Platten auf z.B. Fußboden und oder Wänden. Dabei kann das Reinigungsmittel in flüssiger oder fester Form vorliegen. Der Einsatz kann sowohl als Handgeschirrspülmittel, als auch als Spülmittel für Geschirrspülmaschinen im privaten und oder gewerblichen/industriellen Bereich erfolgen.
Die erfindungsgemäßen stickstoffhaltigen silizium-organischen Pfropfmischpolymere eignen sich ebenfalls zur Verwendung als Wirkstoffe in kosmetischen Zubereitungen oder Körperpflegezusammensetzungen, seien es hautkosmetische Zubereitungen wie Flüssigseifen, Körperlotionen, Rasierwasser, Gesichtswasser, Deodorantien und andere kosmetische Lotionen oder haarkosmetische Zubereitungen, wie Haarkuren, Haarlotionen, Haarspülungen, Haaremulsionen, Spitzenfluids, Egalisierungsmittel für Dauerwellen, Hot-Oil-Treatment-Präparate, Konditionierer, Festigerlotionen, Haarsprays. Darüber hinaus eigenen sie sich für Hand- und Bodylotions, Gesichtsmoisturers, Sonnencreme, Anti-Akne Formulierungen, Anti-Aging Formulierungen, topischen Analgetika, Mascara und ähnliche, wobei die Aufzählung exemplarisch und nicht erschöpfend ist. Je nach Anwendungsgebiet können die kosmetischen Zubereitungen als Spray, Schaum, Gel, Gelspray, Lotion oder Mousse appliziert werden.
Zusätzliche Komponenten, die benötigt werden, um solche Produkte zu formulieren, variieren mit der Produktart und können leicht vom Fachmann gewählt werden, wie Parfümöle, Emulgatoren, Konservierungsmittel, Pflegestoffe wie Panthenol, Collagen, Vitamine, Eiweißhydrolysate, Stabilisatoren, pH-Wert Regulatoren, Farbstoffe, Lösemitteln, Treibgasen und weiteren üblichen und dem Fachmann bekannten Additiven.

Weitere Gegenstände der Erfindung ergehen sich aus den Ansprüchen, deren Offenbarungsgehalt vollumfänglich Gegenstand dieser Beschreibung ist.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Experimentelle Beispiele:

### Beispiel 1:

Herstellung eines stickstoffhaltigen silizium-organischen Pfropfmischpolymer und anschließende Quaternierung.

200 g Siliconpolyether (Tegopren® 5842, Handelsname der Evonik Goldschmidt GmbH, CAS-Nr.: 68937-54-2) wurden in einem Vierhalskolben, ausgestattet mit Rührer, Intensivkühler, Thermometer und Tropftrichter, unter Stickstoffatmosphäre auf 117 °C aufgeheizt. Eine Lösung von 2 g Trigonox® 117 (Handelsname der AkzoNobel, chemischer Name: tert-Butylperoxy-2-ethylhexylcarbonat) in 60 g Dimethylaminoethylmethacrylat wurden innerhalb von 4h zum vorgelegten Siliconpolyether zugetropft. Die Reaktionsmischung wurde anschließend 30 Minuten bei 117 °C gehalten und wurde dann auf Raumtemperatur abgekühlt.
Das Reaktionsprodukt wurde anschließend in 260 g Methoxypropanol gelöst und unter Stickstoffatmosphäre auf 100°C erhitzt. Bei dieser Temperatur wurden 48 g Dimethylsulfat innerhalb von 30 Minuten zugetropft. Die Temperatur soll dabei 105°C nicht überschreiten. Anschließend wurde das Methoxypropanol abdestilliert. Der Rückstand ist das gewünschte Produkt.

### Beispiel 2:

Herstellung eines stickstoffhaltigen silizium-organischen Pfropfmischpolymeres durch radikalische Umsetzung mit einem quaternären Monomer:

80 g Siliconpolyether (Tegopren® 5842, Handelsname der Evonik Goldschmidt GmbH, CAS-Nr.: 68937-54-2) und 40 g Ethanol wurden in einem Vierhalskolben, ausgestattet mit Rührer, Intensivkühler, Thermometer und Tropftrichter, unter Stickstoffatmosphäre vorgelegt und auf 75 °C aufgeheizt. Eine Mischung aus 16 g einer 50%-igen wässrigen Lösung von 3-Trimethylammoniopropylmethacrylamid Chlorid (CAS-Nr.: 51410-72-1), 0,8 g 2,2'-Azobis(2-amidinopropan-dihydrochlorid (Handelsname: V-50 der Wako Pure Chemical Industries, Ltd.) und 20 g Ethanol wurde innerhalb von 4 Stunden zum vorgelegten Siliconpolyether zugetropft. Anschließend wird der Ethanol abdestilliert. Man erhält das gewünschte Produkt als weiße Flüssigkeit.
Es folgen anwendungstechnische Beispiele zum Nachweis der Eigenschaften der erfindungsgemäßen Verbindungen und zum Vergleich dazu Eigenschaften, die mit bekannten Produkten des Standes der Technik erzielt werden können.

### Applikationsbeispiele:

### A) Weichgriff für Textilien:

### Allgemeine Formulierung:

5 bis 50 Gew.% eines stickstoffhaltigen silizium-organischen Pfropfmischpolymeres werden in einem Becherglas mit Propellerrührer unter Rühren vorgelegt. Anschließend werden der Reihe nach 5 bis 25 Gew.-% Dipropylenglykol, 3 bis 10 Gew.-% eines Fettalkoholethoxylates mit einem Ethoxylierungsgrad von 6 und 3 bis 10 Gew.-% eines Fettalkoholethoxylates mit einem Ethoxylierungsgrad von 12 unter Rühren hinzu gegeben. Zuletzt wird mit Wasser auf 100 Gew.-% aufgefüllt.

Analog zur Herstellung der allgemeinen Formulierung wurden aus den Beispielen 1 und 2 die Formulierungen W1 und W2 hergestellt.

### Formulierung W3 - Vergleichsbeispiel:

Eine handelsübliche Mikroemulsion eines mit Aminogruppen funktionalisierten Siloxans, zum Beispiel TEGOSIVIN® IE 11/59 mit einem Feststoffgehalt von 20 Gew.-%.

### Formulierung W4 - Vergleichsbeispiel:

Eine handelsübliche Emulsion eines organischen Weichmachers, zum Beispiel REWOQUAT® WE 18 mit einem Feststoffgehalt von 15 Gew.-%.
Zur Überprüfung des Griffes sowie der Hydrophilie der vorliegenden Erfindung wurden aus nativen Fasern bestehende Produkte mit folgendem Verfahren ausgerüstet:

### Foulardverfahren:

Zur Ausprüfung des Weichgriffs der jeweiligen Emulsionen wurde Baumwollwirkware (160g/m² ) und Baumwoll-Frottierware (400 g/m²) mit einer Flotte, die jeweils 20 g/l der entsprechenden Emulsion enthielt, foulardiert, auf ca. 100 % Flottenaufnahme abgequetscht und bei 130 °C drei Minuten lang getrocknet.

Zur Ausprüfung des Hydrophilie wurden Baumwollwebware (200g/m²) mit einer Flotte, die jeweils 50 g/l der entsprechenden Emulsion enthielt, foulardiert und auf ca. 100 % Flottenaufnahme abgequetscht und bei 130 °C während drei Minuten lang getrocknet.

### Griffbeurteilung:

Zur Beurteilung des Warengriffes wurde ein erfahrenes Team zusammengestellt, das die anonymisierten Griffmuster, der mit den Emulsionen ausgerüsteten Wirk- und Frottierwaren, mit Hilfe eines Handpanneltests bewertete. Bei den Griffmustern aus Maschenware wurde zusätzlich eine nicht offensichtlich gekennzeichnete unbehandelte Probe hinzugelegt.

### Prüfung der Hydrophilie:

Zur Überprüfung der Hydrophilie wurde die an DIN 53924 angelehnte interne Prüfmethode zur Messung der Steighöhe von Wasser verwendet. Dabei wird das ausgerüstete Baumwolltestgewebe in jeweils fünf 25 cm lange und 1,5 cm breite Streifen geschnitten, mit einem wasserlöslichen Stift markiert und an einer Halterung senkrecht straff, aber ohne Spannung, befestigt. Die Halterung wird anschließend für fünf Minuten so in ein Wasserbecken gestellt, dass 2 cm der Streifen ins Wasser eintauchen. Nachdem die Halterung 10 Minuten außerhalb des Wasserbeckens gestanden hat, wird die Steighöhe in cm abgelesen und gegen den Blindwert (Steighöhe der unbehandelten Baumwollstreifen x cm = 100%) bestimmt und in % vom Blindwert angegeben.

### Waschvorgang:

Die Waschvorgänge wurden in einer handelsüblichen Waschmaschine, Miele Novotronic® W 918, mit Buntwäsche ohne Vorwaschen bei 40 °C mit Standardwaschmittel IECA-Base und 3kg BW-Ballastgewebe gewaschen. Zuletzt wurde das so behandelte Gewebe 12 Stunden lang bei Raumtemperatur getrocknet.

Die Weichgriffbeurteilung auf Baumwollwirkware nach Applikation durch Foulard erfolgte vor der Wäsche nach der 1. Wäsche nach der 3. Wäsche und nach der 5. Wäsche.
Die Weichgriffbeurteilung auf Baumwollfrottierware nach Applikation durch Foulard erfolgte vor der Wäsche nach der 1. Wäsche nach der 3. Wäsche und nach der 5. Wäsche.
Die Bestimmung des Rücknetzverhalten auf Baumwollwebware erfolgte vor der Wäsche nach der 1. Wäsche nach der 3. Wäsche und nach der 5. Wäsche.

Es resultiert ein verbesserter Weichgriff bei den mit erfindungsgemäßen Polymeren ausgerüsteten Gewebes im Vergleich zu den mit Produkten des Stand der Technik ausgerüsteten Geweben. Der angenehme Griff bleibt auch nach mehrmaligem Waschen im Wesentlichen erhalten. Zusätzlich ist zu erkennen, dass auch die Hydrophilie, bestimmt über das Rücknetzverhalten, nach mehrmaligem Waschen erhalten bleibt.

### B) Anwendung in der Kosmetik:

### Herstellung und Überprüfung von Haarbehandlungsmitteln:

Für die anwendungstechnische Beurteilung werden Haartressen, die für sensorische Tests verwendet werden, durch eine Dauerwellbehandlung und eine Bleichbehandlung standardisiert vorgeschädigt. Dazu werden friseurübliche Produkte verwendet. Der Testablauf, die verwendeten Basismaterialien sowie die Details der Beurteilungskriterien sind in DE 103 27 871 beschrieben.

### Testformulierungen:

Für die anwendungstechnische Beurteilung werden die erfindungsgemäßen Verbindungen und Vergleichsprodukte in einfachen kosmetischen Formulierungen eingesetzt.
Die Anwendungseigenschaften beim Einsatz in einem Shampoo wurden in der folgenden Rezeptur überprüft:

| Produkt | Gewichtsanteile |
|---|---|
| Natrium Laurylethersulfat (28%ig) z.B. TEXAPON® NSO (Cognis) | 32 |
| "Konditioniermittel" | 0,5 % |
| TEGO^{®} Betain F Cocamidopropyl Betaine (30%) | 10 % |
| Kationisches Polymer zur Verbesserung der Wirksamkeit von Konditioniermitteln (cationic deposition polymer) | 0,3 % |
| (z.B Guar Hydroxypropyl trimonium Chloride, Polyquaternium-10) | |
| Wasser | ad. 100 % |
| Zitronensäure | ad. pH 6,0 ± 0,3 |

Zur Bewertung der Eigenschaften der Shampooformulierung wurde im Testablauf keine Nachbehandlung mit einer Spülung durchgeführt.
Darüber hinaus wurden die erfindungsgemäßen Produkte auch in einer einfachen Haarspülung mit folgendem Aufbau geprüft:

| Produkt | Gewichtsanteile |
|---|---|
| TEGINACID^{®}C | 0, 5 % |
| Ceteareth-25 | |
| TEGO^{®}Alkanol 16 | 2,0 % |
| Cetyl Alcohol | |
| "Konditioniermittel" | 1,0 % |
| VARISOFT^{®} 300 | 3,3 % |
| Cetrimonium Chloride (30%) | |
| Wasser | ad. 100 % |
| Zitronensäure | ad. pH 4,0 ± 0,3 |

Die Vorbehandlung der Haare erfolgt im Falle der Eigenschaftsprüfung von Haarspülungen durch ein Shampoo welches keine Konditioniermittel enthält.

Als "Konditioniermittel" werden die erfindungsgemäßen Verbindungsbeispiele, Vergleichsprodukte oder Kombinationen aus erfindungsgemäßen Verbindungen und bekannten Konditioniermitteln (insbesondere Cetrimonium Chloride) bezeichnet. Als Vergleichsverbindung wurde Quaternium-80 (ABIL® Quat 3272, Handelsname der Evonik Goldschmidt GmbH) eingesetzt.

### Standardisierte Behandlung von vorgeschädigten Haarsträhnen mit konditionierenden Proben:

Die, wie oben beschrieben, vorgeschädigten Haarsträhnchen werden wie folgt mit dem oben beschriebenen Shampoo oder der oben beschriebenen konditionierenden Spülung o behandelt:

Die Haarsträhnen werden unter fließendem, warmem Wasser benetzt. Das überschüssige Wasser wird leicht von Hand ausgedrückt, dann wird das Shampoo aufgebracht und sanft im Haar eingearbeitet (1 ml/Haarsträhne (2 g)). Nach einer Verweilzeit von 1 min wird das Haar für 1 min gespült.
Gegebenenfalls wird direkt im Anschluss die Spülung aufgebracht und sanft im Haar eingearbeitet (1 ml/Haarsträhne (2 g)). Nach einer Verweilzeit von 1 min wird das Haar für 1 min gespült.

Vor der sensorischen Beurteilung wird das Haar an der Luft bei 50 % Luftfeuchtigkeit und 25 °C für mindestens 12 h getrocknet.

Die Zusammensetzung der Testformulierungen ist zur Vermeidung der Beeinflussung der Testergebnisse durch (normalerweise vorhandene) Formulierungsbestandteile bewusst einfach gewählt. Erfindungsgemäße Formulierungen können neben den genannten Inhaltsstoffen und/oder anstatt der genannten Inhaltsstoffe noch weitere Inhaltsstoffe enthalten.

### Beurteilungskriterien:

Die sensorischen Bewertungen erfolgen nach Noten, die auf einer Skala von 1 bis 5 vergeben werden, wobei 1 die schlechteste und 5 die beste Bewertung ist. Die einzelnen Testkriterien erhalten jeweils eine eigene Bewertung.
Die Testkriterien sind: Nasskämmbarkeit, Nassgriff, Trockenkämmbarkeit, Trockengriff, Aussehen/Glanz.

Die Ergebnisse zeigen in überraschender Weise, dass die erfindungsgemäßen Verbindungen bessere Bewertungen erhalten als das Vergleichsprodukt "Quaternium-80".

### Formulierung als Haarspray:

Ein erfindungsgemäßes stickstoffhaltigen silizium-organischer Pfropfmischpolymer, wurde in eine Formulierung für ein Nicht-Aerosol Haarspray mit 80 Massen-% Anteil an flüchtigen organischen Verbindungen (sog. 80% VOC Non-Aerosol Haarspray) nach der in folgender Tabelle aufgeführten Zusammensetzung eingearbeitet.

### Formulierung eines 80% VOC Non-Aerosol Haarspray

| Komponente | Anteil in Gew-% |
|---|---|
| RESYN^{®} 28-2930 Polymer | 5 |
| AMP^{®}-95 | 0,49 |
| silizium-organisches Pfropfmischpolymer | 4,5 |
| ABIL^{®} B 8843 | 0,2 |
| Entionisiertes Wasser | 13,81 |
| SD Alkohol 40 | 80 |

| | |
|---|---|
| RESYN® 28-293 Polymer: (INCI Name: VA/Crotonates/Vinyl Neodecanoate Copolymer) ist ein Produkt der National Starch. AMP®-95: (INCI Name: Amionmethyl Propanol) ist eine Produkt von ANGUS Chemical Company. ABIL® B 8843: (INCI Name: PEG-14 Dimethicone) ist ein Produkt der Evonik Goldschmidt GmbH. SD Alkohol 40: Ethanol. | |

Die Formulierung aus Tabelle zeigte nach Applikation als Haarspray eine verbesserte Flexibilität der behandelten Haare und erzeugte einen fühlbar verbesserten Griff als eine Formulierung, die kein erfindungsgemäßes silizium-organischer Pfropfmischpolymer enthält.

### Formulierung als Haarstylinggel:

Ein erfindungsgemäßes stickstoffhaltiges silizium-organisches Pfropfmischpolymer wurde in eine Formulierung für ein Haarstylinggel nach der in der folgenden Tabelle aufgeführten Zusammensetzung eingearbeitet.

### Formulierung eines Haarstylinggel:

| Komponente | Anteil in Gew.-% |
|---|---|
| AMP^{®}-95 | 0,8 |
| stickstoffhaltiges silizium-organischer Pfropfmischpolymer | 2 |
| Entionisiertes Wasser | 86,4 |
| SD Alkohol 40 | 10 |
| Carbopol^{®} ETD 2020 | 0,8 |

| | |
|---|---|
| AMP-95: (INCI Name: Amionmethyl Propanol) ist eine Produkt von Angus. Carbopol ETD 2020: (INCI Name: Acrylates/C10-30 Alkyl Acrylate Crosspolymer) ist ein Produkt der Firma Noveon. | |

Die Formulierung bildet ein Gel mit einer puddingartigen Konsistenz, welches bei der Applikation als Stylinggel zu einer ausreichende Stabilität im Haar führt bei gleichzeitiger Flexibilität und angenehmen Griffgefühl.

### C) Anwendung in der Autopflege:

Weiterhin ist Gegenstand dieser Erfindung der Einsatz erfindungsgemäßer Verbindungen in der gewerblichen Autowäsche in Trocknungshilfsmitteln in der Autowaschstraße. Der Einsatz erfindungsgemäßer Verbindungen wurde in praxisgerechten Formulierungen überprüft,

### Folgende Basisrezeptur wurde getestet:

| | |
|---|---|
| Butyldiglycol | 8.5 % |
| Dipropylenglycol-butylether | 5.5 % |
| 9-Octadecensaure (Z)-, Reaktionsprodukte mit Triethanolamin, Dimethylsulfat-quaternisiert | 12.0 % |
| Octylpalmitat | 5.0 % |
| Essigsäure | 0.5 % |
| Wasser ad 100 | |

| | |
|---|---|
| Die Basisrezeptur ist Formulierung C1 | |

### Formulierung C2:

Die Basisrezeptur mit 0.8 % Wirkstoff quaternärer Siliconverbindung gemäß Patent EP 294643 ist Formulierung C2.

### Formulierung C3:

Die Basisrezeptur mit 0.8 % Wirkstoff quaternärer stickstoffhaltiger silizium-organischer Pfropfmischpolymere ist Formulierung C3.

Diese Formulierungen wurden praxisgerecht mit Leitungswasser 1 _{:} 1000 verdünnt und die Verdünnungen im Aufrissverhalten untersucht.

Kennzeichnend für die Leistungsfähigkeit ist der Aufriss des Wasserfilms auf dem Autolack wie auch den Glasflächen des Fahrzeuges nach der Aufgabe des Trocknungshilfsmittels. Während eine Bestimmung des Aufrisses auf lackierten Oberflächen schwierig zu reproduzieren ist, sind Glasoberflächen hierzu sehr geeignet.

### Das Aufrissverhalten wurde folgendermaßen bestimmt:

Es wird die Zeit gemessen, die notwendig ist, um einen definierten Wasserfilm auf Glas zu durchdringen und das Glas zu entnetzen. Es wird die erste Reaktionszeit sowie das vollständige Verdrängen des Wassers von einem Objektträger festgehalten.
Spiegelfliese
Objektträger 76x26 mm (3x1 inch)
Pipette 3 ml Kunststoff
Dosierpipette 100 µl
Wasser definierter Qualität; Angabe der Leitfähigkeit
Stoppuhr
Bunsenbrenner

Proben werden gemäß Angabe in Wasser verdünnt vermessen, zumeist in einer 1:1000 Verdünnung. Der Objektträger wird entstaubt und über einer Flamme kurz abgeflammt um eine absolut saubere rückstandsfreie Oberfläche zu gewährleisten.

0,5 ml Wasser werden mithilfe einer Pipette als gleichmäßiger Film auf dem Objektträger aufgetragen. Sollte sich kein Film bilden lassen, muss der Objektträger nochmals gereinigt oder verworfen werden. Anschließend werden 50 µl der Gebrauchsverdünnung der Trocknungshilfe mittig auf die Wasseroberfläche appliziert und die Stoppuhr gestartet. Es werden nun der Beginn der Entnetzung und der Durchbruch des sich zurückziehenden Wassers auf der 26 mm Seite festgehalten. Hierdurch lässt sich die Reaktionszeit und die Aufrissgeschwindigkeit festhalten. Die Angabe erfolgt in Sekunden.

Überraschenderweise zeigen die stickstoffhaltiger silizium-organischer Pfropfmischpolymere enthaltenen Formulierungen eine erhebliche Verkürzung der Aufrisszeit im Vergleich zu literaturbekannten quaternären Siliconverbindungen.

## Patentansprüche

1. Stickstoffhaltige silizium-organische Pfropfmischpolymere, erhalten durch einen radikalischen Pfropfungsschritt mit mindestens einem ethylenisch ungesättigten Monomer, wobei mindestens ein Monomer mindestens eine quaternierbare oder quaternäre stickstoffhaltige Funktionalität enthält, in Gegenwart von polyalkylenoxidhaltigen Siloxanderivaten, die frei von ethylenisch ungesättigten Gruppen sind.

2. Silizium-organische Pfropfmischpolymere nach Anspruch 1, **dadurch gekennzeichnet, dass** die quaternierbare oder quaternäre stickstoffhaltige Funktionalität eine cyclische oder acyclische Aminfunktionalität ist.

3. Silizium-organische Pfropfmischpolymere nach Anspruch 1 oder 2, die mindestens eine permanent quaternäre, positiv geladene Stickstofffunktionalität tragen.

4. Silizium-organische Pfropfmischpolymere nach Anspruch 3, die mindestens eine permanent quaternäre, positiv geladene Stickstofffunktionalität tragen, erhältlich durch die radikalische Pfropfpolymerisation von mindestens einem ethylenisch ungesättigten Monomeren mit mindestens einer quaternären stickstoffhaltigen Funktionalität, in **Gegenwart von** polyalkylenoxidhaltigen Siloxanderivaten.

5. Silizium-organische Pfropfmischpolymere nach zumindest einem der Ansprüche 1 bis 4, **dadurch gekennzeichnet, dass** als Pfropfgrundlage polyalkylenoxidhaltige Polyethersiloxane der Formel (I), 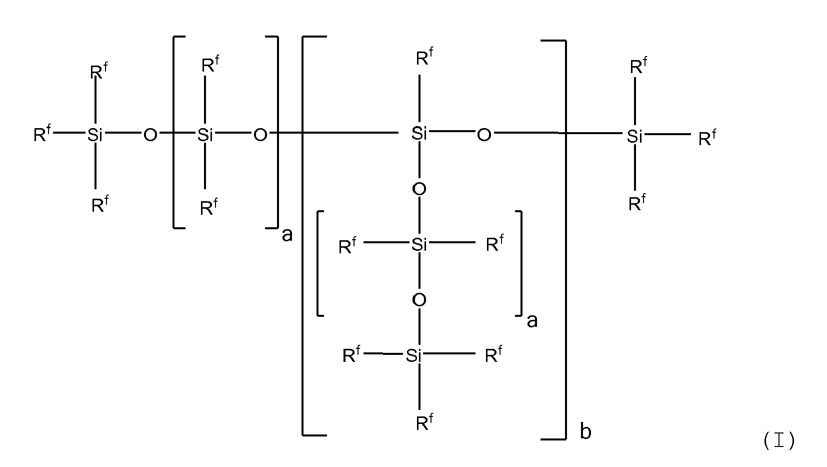 wobei
b eine Zahl von 0 bis 10 ist,
a eine Zahl von 1 bis 500 ist,
R^{f} gleiche oder verschiedene Reste R¹ oder R² sind, mit der Maßgabe, dass mindestens ein Rest R^{f} ein Rest R² ist,
R¹ organische Reste, ausgewählt aus linearen oder verzweigten Alkyl-, Halogenalkyl-, Aryl-, Alkylaryl-oder Arylalkylresten mit 1 bis 30 Kohlenstoffatomen, wobei die Reste gegebenenfalls durch ein oder mehrere Sauerstoff- und/oder Stickstoff-Atome unterbrochen sein können und/oder am Ende des Restes gegebenenfalls eine -OC(O)CH₃ Gruppe aufweisen können,
R² eine Gruppe der Formel A_{α}-B_{β}-K_{χ}-D_{δ}-E_{ε}- L_{λ} darstellt, mit
α 1,
β, χ, δ und ε 0 oder 1,
λ 1 und
α + β + χ ≥ 1, wobei
A ein Sauerstoffatom oder eine CH₂-Gruppe ist,
B eine Gruppe der allgemeinen Formel (II) 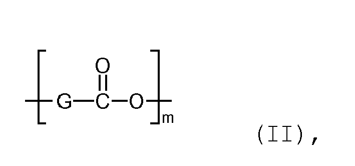 ist, wobei
m eine ganze Zahl von 0 bis 30 ist und
G eine zweiwertige Gruppe, ausgewählt aus linearen oder verzweigten, gesättigten Alkyl-, Aryl-, Alkylaryl-oder Arylalkylgruppen mit 1 bis 20 Kohlenstoffatomen sein kann,
K eine -CH₂-Gruppe oder ein zweiwertiger Rest, ausgewählt aus linearen oder verzweigten, gesättigten Alkyl-, Aryl-, Alkylaryl- oder Arylalkyl-Oxygruppen mit 1 bis 20 Kohlenstoffatomen oder eine Gruppe der Formel -CH₂-O- (CH₂) ₄-O- ist,
D eine Gruppe der allgemeinen Formel (III)
- (C₂H₄O)ₙ(C₃H₆O)ₚ(C₁₂H₂₄O)_{q}(C₈H₈O)ᵣ(C₄H₈O)ₛ- (III)
wobei die Indices n, p, q, r und s voneinander unabhängige ganze Zahlen von 0 bis 100 sind,
und wobei die Summe aus n, p, q, r und s ≥ 1 ist, und falls mehr als einer der Indices n, p, q, r, s > 0 ist, die allgemeine Formel (III) ein statistisches Oligomer oder ein Blockoligomer sein kann,
E eine Gruppe der allgemeinen Formel (IV) 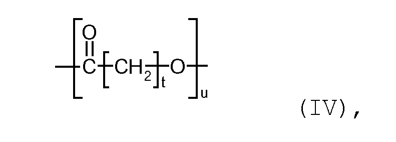
ist, wobei
u eine ganze Zahl von 0 bis 5 ist und
t, falls u > 0 ist, gleich oder verschieden sein kann und eine ganze Zahl 3, 4 oder 5 darstellt und
L ausgewählt ist aus der Gruppe umfassend Wasserstoffatome, lineare oder verzweigte, gesättigte Alkyl-, Aryl-, Alkylaryl- oder Arylalkylgruppen mit 1 bis 12 Kohlenstoffatomen, oder Acetoxy-Gruppen verwendet werden.

6. Silizium-organische Pfropfmischpolymere nach Anspruch 5, **dadurch gekennzeichnet, dass** als Pfropfgrundlage Polyethersiloxane der Formel (I) n mit A = -CH₂-, α = 1, β = 0, χ = 1 verwendet werden.

7. Silizium-organische Pfropfmischpolymere nach Anspruch 6, **dadurch gekennzeichnet, dass** als Pfropfgrundlage Polyethersiloxane der Formel (I) mit zusätzlich K = -CH₂-CH₂-O- verwendet werden.

8. Silizium-organische Pfropfmischpolymere nach zumindest einem der Ansprüche 1 bis 4, **dadurch gekennzeichnet, dass** als ungesättigte Verbindung für die Pfropfpolymerisation mindestens eine monomere, ethylenisch ungesättigte Verbindung und polymere Olefine oder Macromonomeren mit mindestens einem Rest an Ungesättigtkeit einschließlich solcher die Siloxanketten enthalten und mindestens eine stickstoffhaltige Funktionalität besitzt, die quaterniert werden kann, verwendet werden.

9. Silizium-organische Pfropfmischpolymere nach Anspruch 8, **dadurch gekennzeichnet, dass** Mischungen von stickstoffhaltigen und stickstofffreien Monomeren pfropfpolymerisiert werden.

10. Silizium-organische Pfropfmischpolymere nach Anspruch 9, **dadurch gekennzeichnet, dass** zusätzlich zu mindestens einem Monomer mit mindestens einer stickstoffhaltigen Funktionalität als weitere ungesättigte stickstofffreie Verbindung in der Pfropfpolymerisation Verbindungen der allgemeinen Formel (VII) verwendet werden 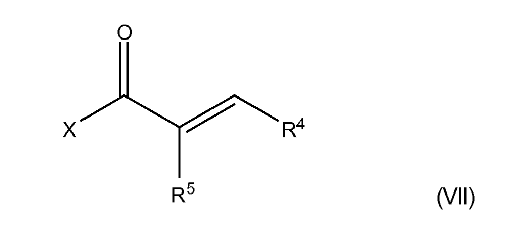 wobei R⁵ und R⁴ unabhängig voneinander ausgewählt sind aus der Gruppe enthaltend: -H, C₁-C₈ linear- oder verzweigtkettige Alkylketten, Methoxy, Ethoxy, 2-Hydroxyethoxy, 2-Methoxyethoxy und 2-Ethoxyethyl,
X ist ausgewählt aus der Gruppe enthaltend die Reste - OH, -OM, -OR⁶, NH₂, -NHR⁶, N (R⁶) ₂, wobei die Reste R⁶ identisch oder verschieden sein können und ausgewählt sind aus der Gruppe bestehend aus -H, C₁-C₄₀ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl,
M ist ein Kation ausgewählt aus der Gruppe bestehend aus: Na⁺, K⁺, Mg⁺⁺, ca⁺⁺, Zn⁺⁺ NH₄⁺, Alkylammonium, Dialkylammonium, Trialkylammonium und Tetraalkylammonium.

11. Silizium-organische Pfropfmischpolymere nach Anspruch 1 bis 3 und 8 bis 10, **dadurch gekennzeichnet, dass** als stickstoffhaltige Monomere N,N-Dialkylaminoalkylacrylat und -methacrylat und N-Dialkylaminoalkylacryl- und -methacrylamid der allgemeinen Formel (VIII) 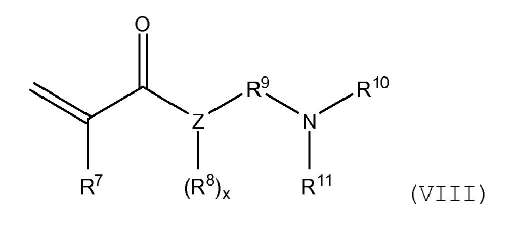 mit R⁷ = H, Alkyl mit 1 bis 8 C-Atomen, R⁸ = H, Methyl, R⁹ = Alkylen mit 1 bis 24 C-Atomen, optional substituiert durch Alkyl, R¹⁰ und R¹¹ unabhängig voneinander gleich C₁-C₄₀ Alkylrest, Z = Stickstoff für X = 1 oder Sauerstoff für x = 0, wobei die Amide unsubstituiert, N-Alkyl oder N-alkylamino monosubstituiert, oder N,N-dialkylsubstituiert oder N,N-dialkylamino disubstituiert, worin die Alkyl- oder Alkylaminogruppen von C₁-C₄₀ linearen, C₃-C₄₀ verzweigtkettigen, oder C₃-C₄₀ carbocyclischen Einheiten abgeleitet sind, sein können, verwendet werden.

12. Silizium-organische Pfropfmischpolymere nach Anspruch 11, **dadurch gekennzeichnet, dass** die Alkylaminogruppen quaternisiert sind.

13. Silizium-organische Pfropfmischpolymere nach Anspruch 11 oder 12, **dadurch gekennzeichnet, dass** die stickstoffhaltigen Monomere mindestens eine quaternäre stickstoffhaltige Gruppe aufweisen und der allgemeinen Formel (XI) entsprechen, mit 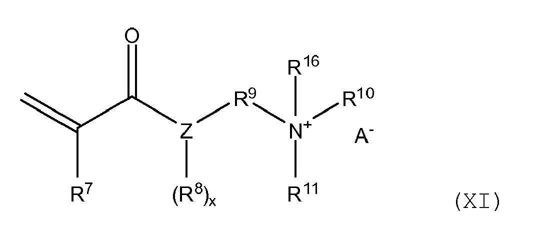 mit der Bedeutung von R⁷, R⁸, R⁹ ,R¹⁰, R¹¹ , ,Z und x wie in Formel (VIII), R¹⁶ = C₁-C₄₀ Alkylrest und A⁻ ein geeignetes negativ geladenes Anion ist, ausgewählt aus der Gruppe Fluorid, Chlorid, Bromid, Jodid, Alkylsulfate, Methylsulfat oder Ethylsulfat, Sulfat, Hydrogensulfat, Methansulfonat, Trifluormethansulfonat.

14. Silizium-organische Pfropfmischpolymere nach Anspruch 13, **dadurch gekennzeichnet, dass** als Verbindungen der Formel (XI) 2-Trimethylammoniumethylmethacrylatchlorid, 2-Trimethylammoniummethylacrylatchlorid, 2-Triethylammoniumethylmethacrylatchlorid, 2-Triethylammoniumethylacrylatchlorid, 3-Trimethylammoniumpropylmethacrylamidchlorid, 3-Trimethylammoniumpropylacrylamidchlorid, 3-Triethylammoniumpropylmethacrylamidchlorid, 3-Triethylammoniumpropylacrylamidchlorid, verwendet werden.

15. Zusammensetzungen enthaltend Pfropfmischpolymere nach zumindest einem der Ansprüche 1 bis 14.

16. Verwendung der stickstoffhaltigen silizium-organischen Pfropfmischpolymere oder Zusammensetzungen gemäß zumindest einem der Ansprüche 1 bis 15 als Weichmacher oder Weichgriffmittel für Gewebe, Non-wovens und/oder Fasern aus natürlichen und/oder synthetischen Rohstoffen, für Textilien.

17. Verwendung der stickstoffhaltigen silizium-organischen Pfropfmischpolymere oder Zusammensetzungen gemäß zumindest einem der Ansprüche 1 bis 15 als Additiv in kosmetischen Anwendungen, als Bestandteil von Haarbehandlungsmitteln in Form von Shampoo, Haarspülung, Konditioniermittel, Haarspray, und/oder als Bestandteil von Haarstylinggel.

18. Verwendung der stickstoffhaltigen silizium-organischen Pfropfmischpolymere oder Zusammensetzungen gemäß zumindest einem der Ansprüche 1 bis 15 für die Reinigung und Pflege harter Oberflächen, harten lackierten oder unlackierten Oberflächen aus Glas, Keramik, Kunststoff oder Metall, und von Geschirr, in Haushalt und Gewerbe, s o wie in der gewerblichen Autowäsche in Trocknungshilfsmitteln in Autowaschstraßen.
